Europäisches Patentamt

European Patent Office

Office européen des brevets

(11) Publication number: **0 074 279**
**B1**

# EUROPEAN PATENT SPECIFICATION

(45) Date of publication of patent specification: **12.08.87**

(21) Application number: **82304730.3**

(22) Date of filing: **08.09.82**

(51) Int. Cl.⁴: **A 61 K 39/395,** A 61 K 47/00 // C12N15/00, C12N5/00, C12P1/00, C12R1/91

(54) Selective anti-tumour agents.

(30) Priority: **08.09.81 JP 142158/81**

(43) Date of publication of application:
**16.03.83 Bulletin 83/11**

(45) Publication of the grant of the patent:
**12.08.87 Bulletin 87/33**

(84) Designated Contracting States:
**IT**

(56) References cited:
EP-A-0 017 507
EP-A-0 023 401
EP-A-0 044 167
EP-A-0 055 575
EP-A-0 063 988

BIOLOGICAL ABSTRACTS, vol. 71, no. 7, 1981, page 4909, no. 46810, Philadelphia, US D.G. GILLILAND et al.: "Antibody-directed cytotoxic agents: Use of monoclonal antibody to direct the action of toxin A chains to colorectal carcinoma cells"

(73) Proprietor: **Suntory Limited**
**1-40 Dojimahama 2-chome Kita-ku**
**Osaka-shi Osaka-fu 530 (JP)**

(72) Inventor: **Nakanishi, Toshihiro**
**No. 8, Suntory Shataku No. 16-16, Futaba-cho**
**Ibaragi-shi Osaka (JP)**
Inventor: **Yoshizumi, Hajime**
**55A-36-208, No. 115 Nobori-Cho**
**Takatsuki-shi Osaka (JP)**
Inventor: **Imai, Kozo**
**Higashi 2-chome Kita 16-jo Higashi-ku**
**Sapporo-shi Hokkaido (JP)**
Inventor: **Yachi, Akira**
**Nishi 14-chome Minami 14-jo Chuo-ku**
**Sapporo-shi Hokkaido (JP)**
Inventor: **Soldano, Ferrone**
**No. 8936, Scenic Drive North**
**La Jolla California, 92307 (US)**

(74) Representative: **Moore, Anthony John et al**
**Gee & Co. Chancery House Chancery Lane**
**London WC2A 1QU (GB)**

# 0 074 279

## Description

This invention relates to selective anti-tumour agents in which a monoclonal antibody to a specific antigen of human tumour is conjugated with a voluntary anti-tumour substance, so that the conjugate selectively attacks specific tumour tissues or tumour cells as its target when administered to tumour patients.

Heretofore, numerous chemotherapeutic anti-tumour agents have been studied and applied clinically but they have comparatively low reliability since such chemotherapeutic agents are toxic to normal cells as well as to tumour cells and it is therefore impossible to administer them to the patients in sufficient amounts.

Because of this considerable efforts have been made to discover selective anti-tumour agents by using special antibodies as carriers which selectively transport the anti-tumour substances to the tumour cells or tissues; see, for example, Japanese published patent application No. 144723/'76, J. Natl. Cancer Inst., *55*, 473—477 (1975), and Nature, *271*, 752—754 (1978).

However, the above carriers are not specific antibodies to the tumour antigen. Therefore, they are insufficiently selective and possibly injure normal cells at a high rate.

According to the invention we provide a selective anti-tumour agent comprising an anti-tumour active substance chemically conjugated with an antibody as a carrier, characterised in that the anti-tumor active substance is a cytotoxic basic polypeptide $SP_1$, $SP_2$ or SP-H derived from barley or wheat and the antibody is a monoclonal antibody to the tumor antigen.

The specific antibody can be prepared from the propagates of a fused cell (hybridoma) caused by cell-fusion between an animal cell (e.g., a spleen cell from BALB/c mouse) sensitized by a human tumour cell (such as a malignant melanoma cell) and another animal cell (e.g., a myeloma cell from a mouse). The above propagates are subjected to cloning whereby special hybridomas, capable of producing monoclonal antibody (MoAb), which selectively connects to specific antigen of human tumour, are formed and then the desired MoAb is isolated from such hybridomas. The anti-tumor basic polypeptide has a strong cytotoxic property.

The conjugation between the anti-tumour substances and the above-mentioned monoclonal antibody (MoAb) is carried out by chemically combining the free amino or carboxyl moiety of the latter with, for example, an amino, carboxyl or aldehyde moiety, as follows:

Anti-tumour substances which are proteins or polypeptides. The major proportion of amino groups is guanidilated and the remaining free amino groups are then condensed with the carboxyl group of the acidic amino acid moieties of the antibody using carbodiimide reagent. This method commonly gives good results with basic polypeptides of barley or wheat ($SP_1$, $SP_2$, SP-H).

The invention will now be illustrated by the following Examples which are given with reference to the accompanying drawings, in which

Figure 1 is an elution curve showing fractionation of a SP-H · antibody conjugation-reaction product through Sephadex® G-200;

Figure 2 is a graph showing selective absorption property of SP-H · MoAb conjugate to Colo 38 and Raji cells;

Figure 3 and Figure 4 are each a graph showing inhibition effect of SP-H · MoAb conjugate against Colo 38 cells (melanoma cells) and Raji cells (normal cells), respectively;

Figure 5 and Figure 6 are each a graph showing tymidine-intake inhibition effect of SP-H · MoAb conjugate against Colo 38 cells and Raji cells, respectively;

Figure 7 and Figure 8 are each a graph showing the effect of SP-H · MoAb conjugate on the survival of cancerized mice.

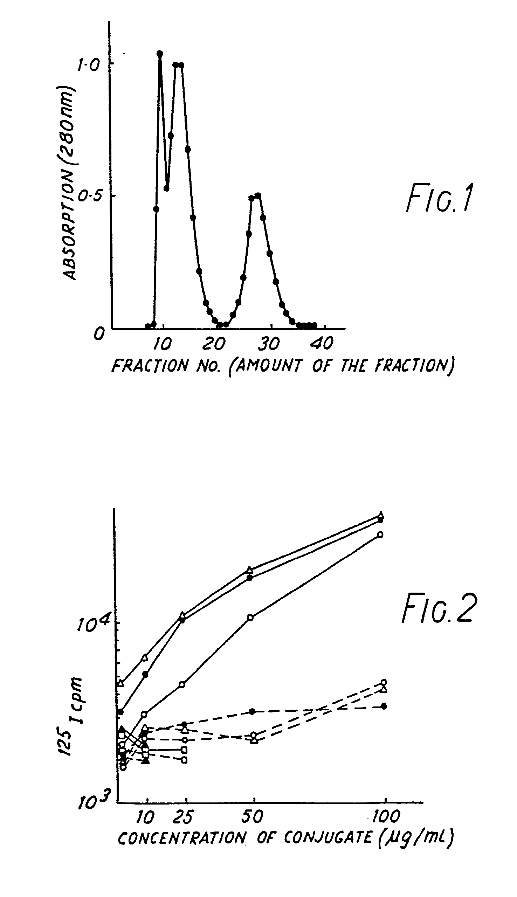

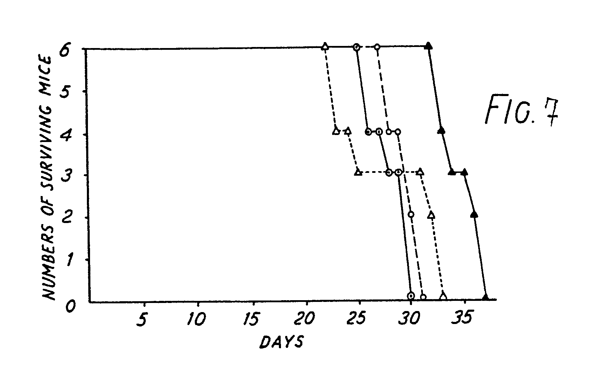

Throughout Figures 2 to 6, straight lines show the effect on Colo 38 and dotted lines show the effect on Raji cells; and symbols △, ●, ○, ▲ and □ show MoAb alone, conjugate I (F-I in Figure 1), conjugate II (F-II in Figure 1), Fraction III (F-III) in Figure 1 and SPH alone (Substance No. SUN 9102), respectively. Symbols ▲, △, ⊙ and ○ in Figure 7 and Figure 8 show SP-H · MoAb conjugate, MoAb alone, non-treatment and SP-H alone, respectively.

Example 1
Cultivation of hybridoma (225, 28s) and purification of MoAb

Cells of hybridoma 225, 28s preliminarily cultivated in Dulbecco's Modified Eagle medium containing 10% calf serum were inoculated intrapenetreally into BALB/c mice cells into which 0.3 ml of pristeine (2,6,10,14-tetramethyl pentadecane) had been injected intrapenetreally one week before the inoculation. (Aged 8—12 weeks, each $5\times10^6$).

The penetral water of the living mice was collected after one week had elapsed from the inoculation of the hybridoma. The collected penetreal water was then centrifuged at 3000 rpm for 10 minutes in order to remove leucocytes and pristeine. To the supernatant, there was added saturated ammonium sulfate solution until the final concentration reached 33.3% and it was then allowed to stand for one hour at 4°C. The precipitate formed was collected centrifugally (10,000 rpm, 10 minutes, 4°C). The precipitate was then dissolved in a small amount of phosphate buffer saline from which $Ca^{++}$ and $Mg^{++}$ were absent (PBS) and dialysed overnight for PBS with a dialysing tube (Cellophane Tubing-Seamless, Union Carbide, U.S.A.).

2

**0 074 279**

The outer liquid was replaced at least 4 times during the dialyses and finally dialysed for M/100 tris-HCl buffer (pH 8.0) and then loaded on DE-52 column (2.5×16.5 cm), buffered with equal buffer solution. This column was eluted with the same buffer but so that the NaCl concentration in the buffer changes linearly from 0 to 0.5 M under the addition of NaCl. MoAb activity in the eluted peak fraction (at 280 nm) was measured with $^{125}$I-protein A by the amount of binding to human malignant melanoma. The active fraction was concentrated at 4°C and then passed through Sephadex® GWO column (1.5×90 cm). Thus, the fraction was purified up to unitary peak at 280 nm. This peak fraction was unitary even after electrophoresis and could be stored by freeze-drying or under freeze conditions. Moreover, the foregoing cultivation, purification and isolation techniques are applicable for other MoAb to human malignant melanoma, for example 376.74, 376.96, 465.14 and 653.25. Each antibody specifically combined with each corresponding antigen of the melanoma, but the binding portion is somewhat different depending on the kind of each antibody. Thus, the fact of these various combinations suggests a possibility for a cocktail therapy by conjugates between several different MoAb and anti-tumour agents.

Example 2
Preparation of first therapeutic MoAb conjugate

2.582 g (0.5 mol) of O-methylisourea were dissolved in 25 ml of water. The solution was adjusted to pH 3.2 with 10N NaOH. 300 mg of SP-H (which is a basic polypeptide isolated from barley by the present inventors and constituted of 45 amino acids cross-linked through 4-S-S-linkages as described in Japanese Published Patent Application No. 49342/'79) was added to the above solution and dissolved. The solution thus formed was further adjusted to pH 9.2 with the further addition of NaOH solution and allowed to stand for 48 hours at 4°C. The reaction mixture was next subjected to gel-filtration through Sephadex® G-10, and the eluate freeze-dried so as to give 299 mg of guanidilated SP-H. In this sample, there were about four free amino radicals in the ε-lysines in the original SP-H but cytotoxic activity against tumour cells was almost unchanged. 35 mg of the above guanidilated SP-H and 10 mg of freeze-dried antibody obtained by the Example 1 were dissolved in 1.5 ml of PBS. To the solution 60 µl of aqueous solution of water-soluble carbodiimide were added and adjusted to pH 8.5 with 6N HCl and stirred for 4 hours at room temperature. The reaction mixture was then immediately subjected to gel-filtration with a Sephadex® G-200 column (1.5×90 cm). The column was eluted with PBS at a velocity of 24 ml/hour and the eluate fractionated into 4 ml fractions. As shown by Figure 1, the eluate was fractionated into three fractions under the parameter of absorption at 280 nm. As discussed below, the fraction F-I (tube Nos. 9.10) and F-II (tube Nos. 12—15) are desired SP-H · MoAb conjugates. On the other hand, the fraction F-III (tube Nos. 26—30) was perhaps unreacted SP-H.

Immunological properties of the conjugates

The therapeutic MoAb conjugates thus obtained were selectively combined with target tumour cells as follows:—

a) Materials
Cells: Colo 38 cells (human malignant melanoma cells) and Raji cells (human lymphoid cells derived from B-cells).

Culture media
For Colo 38:D-MEM (hereinbefore described) For Raji:RMP 11640 with 10% calf serum Medicine: SP-H · MoAb conjugate according to Example 2. The medicine was applied as PBS solution and respective further diluted culture media.

b) Experimental method
Colo 38 and Raji cells were preincubated for three days. The propagated cells were washed five times with the respective culture medium and poured into plastic plates so as to transfer $10^5$ cells to the respective plate. To each plate, 0—100 µg/ml (final concentration) of the conjugate were added and allowed to stand for one hour under stirring. Then, the cells were washed five times similarly, and then an appropriate amount of protein A (which was derived from *Staphylococcus aureus* sp.) marked with $^{125}$I was added to the cells and allowed to stand for one hour. Then, the cells were again washed five times and the amount of $^{125}$I adhered to the cells measured with Gamma counter (by Beckmann, USA). The protein A combined with the Fc portion of the conjugate.

c) Results
As shown by Figure 2, MoAb (225.28s), conjugate I (F-I) and conjugate II (F-II) Colo 38 cells depend on their concentration, but Raji cells which have no adaptable antigen never combine with any antigen (225, 28s), conjugate I (F-I) or conjugate II (F-II). Fraction F-III (presumably unreacted SP-H) and SP-H alone naturally do not combine with either Colo 38 or Raji cells.

Cytotoxic effect of the conjugates
a) Materials: As above.

b) Method

Each $2\times10^5$ per ml of cells were dispersed into each plate and 0—100 µl of the conjugate added into the plate followed by incubation at 37°C for 48 hours. Then the cells were sampled from each plate and stained with tripane blue. The life or death of the cells was judged by the stainability (stained: deaded; unstained: alive) and counted by a Tuerk-Buerker counting disk.

c) Results

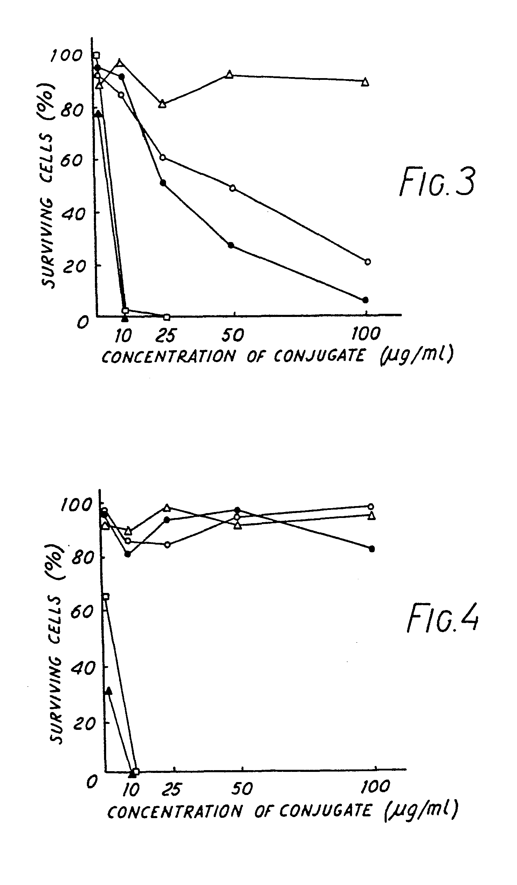

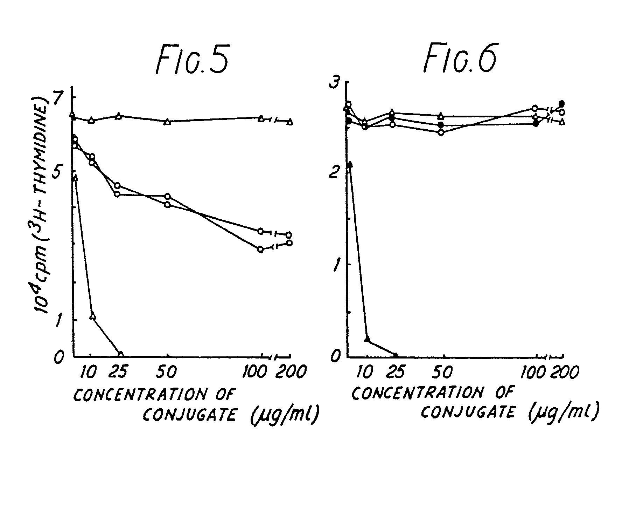

As shown by Figures 3 and 4, conjugates I and II show cytotoxic activity against Colo 38 cells having melanoma antigen (cf. Figure 3), but they are almost inactive against Raji (cf. Figure 4). On the contrary, MoAb (225.28s) does not affect any cells but medicine (SP-H) alone and fraction F-III exert strong toxicity to all cells.

Anti-tumour effect of the conjugates on experimental animals

a) Materials

Cells: Colo 38

Culture medium: RMPI 1640 containing 10% calf serum

Medicine: SP-H · MoAb conjugate obtained according to Example 2

Animals: BALB/cA (-nu/JCR) nude mice, female, aged 6 weeks.

b) Method

Melanoma cells were inoculated intrasubcutaneously into each mouse. The medicine and controls (antibody alone and SP-H alone) were intrapenetreally administered into the animals in amounts of 0.1 mg/mouse, 1 mg/mouse and 0.5 mg/Kg at 1, 3, 5 and 20 days after the inoculation.

c) Results

The results are shown in Figure 7. The mice of the control group (each constituted by six mice) were dead within 30 days: i.e., neither SP-H alone or antibody alone could prolong the life of the tested mice. On the contrary, the medicine-administered group (each constituted by six mice) clearly survived for 37 days after the inoculation.

The MoAb conjugates according to the present invention exert strong affinity to specific tumour cells having corresponding antigen and kill said cells or inhibit their growth, but do not affect normal cells. Therefore, it can be expected that the new conjugate contributes to the chemotherapy of many malignant tumours or cancers.

**Claims**

1. A selective anti-tumor agent comprising an anti-tumor active substance chemically conjugated with an anti-body as a carrier, characterised in that the anti-tumor active substance is a cytotoxic basic polypeptide $SP_1$, $SP_2$ or SP-H derived from barley or wheat and the antibody is a monoclonal antibody to the tumor antigen.

2. An agent as claimed in Claim 1, wherein the monoclonal antibody is produced by hybridoma.

3. An agent according to Claim 1, wherein the hybridoma is a fused cell formed by cell fusion between an animal cell sensitized by a human tumor cell and an animal myeloma cell.

4. An agent according to Claim 3, wherein said animal cell is a spleen cell.

5. An agent according to Claim 3 or 4, wherein said human tumour cell is a malignant melanoma cell.

6. An agent according to any preceding claim, wherein carboxyl residues of acidic amino acid groups in the antibody and amino radicals of ε-lysine in the anti-tumor active substance are combined to form amidic links.

**Patentansprüche**

1. Selektives Antitumormittel bestehend aus einem chemisch mit einem Antikörper als Träger konjugierten antitumoralen Wirkstoff, dadurch gekennzeichnet, dass der antitumorale Wirkstoff ein von Gerste oder Weizen abgeleitetes zytotoxisches basisches Polypeptid $SP_1$, $SP_2$ oder SP-H und der Antikörper ein monoklonaler Antikörper für das Tumorantigen ist.

2. Mittel nach Anspruch 1, worin der monoklonale Antikörper durch ein Hybridom erzeugt wird.

3. Mittel nach Anspruch 1, worin das Hybridom eine durch Zellfusionierung zwischen einer durch eine menschliche Tumorzelle sensibilisierten tierischen Zelle und einer tierischen Myelomzelle gebildete fusionierte Zelle ist.

4. Mittel nach Anspruch 3, worin besagte tierische Zelle eine Milzzelle ist.

5. Mittel nach Anspruch 3 oder 4, worin besagte menschliche Tumorzelle eine bösartige Melanomzelle ist.

6. Mittel nach einem der vorhergehenden Ansprüche, worin Carboxylreste von sauren Aminosäuregruppen im Antikörper und Aminoreste von ε-Lysin im antitumoralen Wirkstoff unter Bildung von Amidverknüpfungen miteinander verbunden sind.

# 0 074 279

**Revendications**

1. Un agent anti-tumoral sélectif comprenant une matière active anti-tumorale, conjuguée chimiquement avec un anticorps en tant que support, caractérisé en ce que la matière active anti-tumorale est un polypeptide basique cytotoxique SP$_1$, SP$_2$ ou SP-H dérivé de l'orge ou du blé et en ce que l'anticorps est un anticorps monoclonal de l'antigène tumoral.

2. Un agent selon la revendication 1, dans lequel l'anticorps monoclonal est produit par un hybridome.

3. Un agent selon la revendication 1, dans lequel l'hybridome est une cellule fusionnée, formée par fusion cellulaire entre une cellule animale sensibilisée par une cellule tumorale humaine et une cellule de myélome d'origine animale.

4. Un agent selon la revendication 3, dans lequel ladite cellule animale est une cellule de rate.

5. Un agent selon la revendication 3 ou la revendication 4, dans lequel ladite cellule tumorale humaine est une cellule de mélanome malin.

6. Un agent selon l'une quelconque des revendications précédentes, dans lequel les restes carboxyles des groupes acides des acides aminés dans l'anticorps et les radicaux amino de la lysine-ε dans la matière active anti-tumorale sont combinés pour former des liaisons amidiques.

FIG.1

FIG.2

FIG. 3

FIG. 4

FIG.5

FIG.6

$10^4$ cpm ($^3$H-THYMIDINE)

CONCENTRATION OF CONJUGATE (μg/ml)

CONCENTRATION OF CONJUGATE (μg/ml)

FIG. 7